Europäisches Patentamt

European Patent Office

Office européen des brevets

(11) Publication number: **0 303 294**
**A2**

(12) **EUROPEAN PATENT APPLICATION**

(21) Application number: **88113173.4**

(51) Int. Cl.⁴ **C12M 3/00**

(22) Date of filing: **12.08.88**

(30) Priority: **14.08.87 JP 202817/87**

(43) Date of publication of application:
**15.02.89 Bulletin 89/07**

(84) Designated Contracting States:
**CH DE GB LI SE**

(71) Applicant: **MITSUBISHI KASEI CORPORATION**
**5-2, Marunouchi 2-chome Chiyoda-ku**
**Tokyo(JP)**

(72) Inventor: **Kusano, Hiroshi**
**1-24-14 Fujigaoka Midori-ku**
**Yokohama-shi Kanagawa-ken(JP)**
Inventor: **Ito, Tsuyoshi**
**1-8-20 Ogawa**
**Machida-shi Tokyo(JP)**
Inventor: **Kubota, Hirohisa**
**2-6-3 Tachibanadai Midori-ku**
**Yokohama-shi Kanagawa-ken(JP)**

(74) Representative: **Patentanwälte TER MEER -**
**MÜLLER - STEINMEISTER**
**Mauerkircherstrasse 45**
**D-8000 München 80(DE)**

(54) **Cell culture filmcarriers.**

(57) Improved cell culture filmcarriers are disclosed herein. These improved filmcarriers consist of water-insoluble polymer film, the film being 1.0 - 2,000 μm in thickness and 1.00 - 1.30 g/ml in density in a culture medium and having positively chargeable chemical moieties or proteins, which are linked on the film surface through at least chemical bonds.

EP 0 303 294 A2

## CELL CULTURE FILMCARRIERS

BACKGROUND OF THE INVENTION

1.Field of the Invention

This invention relates to cell culture filmcarriers for use in the culture of animal cells, particularly to cell culture filmcarriers suitable for use in the high-density cultivation of anchorage-dependent animal cells or hybridomas (referred to hereinafter only as "the cells") attached to the surface of suspended or stationary filmcarriers.

2.Description of the Prior Art

In recent years, a rapid progress has been made in the culture of animal cells. One of the previous operational techniques employed therefor is a microcarrier system. The microcarriers which have been reported to date are, for example, spherical ones formed from a cross-linked dextran matrix such as Cytodex®(commercially available from Pharmacia Fine Chemicals, Inc.,) and Superbeads®(commercially available from Flow Labs, Inc.,), spherical ones made of polystylene (about 100 - 200 $\mu$m in diameter) such as Biosilon (commercially available from Nunc Inter. Med.) and Cytosphere (commercially available from Lux Sci. Co.), cellulose particles such as DE-52 and DE-53 (commercially available from Whatman, Inc.,), and filamentary ones such as a hollow fiber. These spherical or filamentary carriers,however, have a disadvantage that their surface area per volume is so small that the cells may not attach themselves equally thereover. This means that the surface area can not used efficiently. Furthermore, it is known that the cells may recognize the radius of curvature of microcarriers so that their attachability to the microcarriers or distensibility and growth capacity will be deleteriously effected when the size of the cells are relatively large to the diameter of the microcarriers.

Thus, an aspect of the present invention is to provide cell culture filmcarriers which can be produced economically.

Another aspect of the present invention is to provide cell culture filmcarriers whose surface area per volume is larger than that of conventional microcarriers so that the surface area may be efficiently utilized by the cells in the high-density cultivation.

A further aspect of the present invention is to provide cell culture filmcarriers having a substantially indefinite radius of curvature for the cells, which can allow the cells to show a good attachability, distensibility and growth capacity.

A still further aspect of the present invention is to provide cell culture filmcarriers which may permit the culture cells to economically produce biologically active substances such as a vaccine, enzyme and protein in a large scale.

The cell culture filmcarriers according to the present invention consist of water-insoluble polymer film, the film being 1.0 -2,000$\mu$m in thickness and 1.00 - 1.30 g/ml in density in a culture medium and having positively chargeable chemical moieties or proteins, which are linked on the film surface through at least chemical bonds.

DETAILED DESCRIPTION OF THE INVENTION

The essential characteristics of the present invention may reside in the fact that a base matrix of the filmcarriers according to the present invention consists of water-insoluble polymer film the film being 1.0 - 2,000 $\mu$m in thickness and 1.00 - 1.30 g/ml in density in a culture medium and having positively chargeable chemical moieties or proteins,which are linked on the film surface through at least chemical bonds.

The above positively chargeable chemical moieites may be preferably ammonia or amine having 5 or less carbon atoms. Any water-insoluble polymer may be used in the present invention, the polymer constituted by (meth)acrylic ester or (meth)acrylic amide being preferable and that containing at least (meth)acrylic ester as a monomer unit being more preferable.

The filmcarriers according to the present invention may be prepared as follows:

A mixture of a monomer solution and a polymerization initiator is poured into a cell consisting of a pair of glass plates spaced from each other through a spacer followed by polymerization to give a film gel. The monomer subjected to the above preparation may be selected in accordance with a kind of the desired polymer film.

As the monomer suitable for use in the present invention, there may be exemplified (meth)acrylic esters such as 2-hydroxyethyl (meth)acrylate, diethyleneglycol (meth)acrylate, octaethyleneglycol (meth)acrylate, polyethyleneglycol (meth)acrylate, methyl (meth)acrylate, ethyl (meth)acrylate and glycidyl (meth)acrylate. Any suitable cross-linking agent may be optionally added to the above ester monomers. The polymer film constituted by polyethyleneglycol (meth)acrylate and/or glycidyl (meth)acrylate may be preferably prepared in the presence of the cross-linking agent in order to prevent the leakage of the (meth)acrylate ester from the film thus produced and to increase a mechanical strength thereof unless the polymer film prepared without the cross-linking agent is water-insoluble. Any strongly hydrophilic monomer which can be co-polymerized with polyethyleneglycol (meth)acrylate and/or glycidyl (meth)acrylate may be used as the cross-linking agent, examples of which are ethyleneglycol poly(meth)acrylate, glycerol poly(meth)acrylate.

Although the addition of the cross-linking agent is preferable in view of the above reasons, a high content thereof would adsorb proteins in a serum and biologically active substances including antibodies secreted by the cells and would therefore reduce a recovering rate of such substances. Accordingly, the content of the cross-linking agent preferably ranges from 0 to 90 %, more preferably from 10 to 60 % by weight.

2-Hydroxyethyl (meth)acrylate, polyethyleneglycol (meth)acrylate, glycidyl (meth)acrylate and the like may be advantageously used in the present invention because they can economically prepare the hydrophilic polymer film.

The suspension polymerization of O/W or W/O/W type may be performed in a conventional manner in the presence of an organic diluent and/or polymerization initiator.

Any organic diluent which is capable of dissolving the monomers and inactive to functional groups such as glycidyl and hydroxyl may be used for the polymerization. Examples of the suitable organic diluent included benzene, toluene, chlorobenzene, methylisobutylketone, 1-hexanol, cyclohexanol, 1-decanol and 1,2-dichloroethane and butyl acetate. The organic diluents may be preferably added in 0 - 10 times by weight the total amount of the monomers. The use of too much amount of organic diluent would reduce the degree of polymerization and even make the resulting polymer film fragile.

The monomer with a high content of an oxyethylene group may easily yield a transparent film gel·as far as the same organic diluent is used.

Either filmcarriers in the form of a porous film having fine pores or filmcarriers punched upto 0.1 - 30% of their surface area may be used in the present invention as long as their mechanical strength and contact surface area with the cells are never decreased.

Examples of suitable polymerization initiators include benzoyl peroxide, lauroyl peroxide and azobisisobutyronitrile, which may be usually used in an amount of from 0.01 to 5 %, preferably from 0.05 to 1.0 % by weight of the total monomers. The polymerization may be initiated by the irradiation of ultraviolet.

The density of the cell culture filmcarriers according to the present invention may depend on the composition of the monomers, the ratio of the cross-linking, the type and used amount of the organic diluent, the charge capacity of amines, and the size and thickness of the polymer film. The density of the filmcarriers should be, however, preferably adjusted to the ranged of from 1.00 to 1.30 g/ml in order to keep the filmcarriers homogeneously suspended under stirring or in a flowing state (an air-lift or up-flow type) during cultivation. Furthermore, the filmcarriers preferably range from 1 to 2,000 μm in thickness and extend over $10^{-3}$ to $10^1$ cm$^2$ per a sheet of the film. It is further desired that the size of each sheet of the film should hardly vary in order to effect the homogeneous attachment and growth of cells as well as the homogeneous suspension of the filmcarriers.

The existence of the positively chargeable chemical mopieties or proteins on the surface of the filmcarriers is inevitably required in order that the filmcarriers may attach the cells thereon so as to proliferate and culture them even in a serum-free medium.

As previously described, ammonia or amine having 5 or less carbon atoms may be advantageously employed as the positively chargeable chemical moieties by the following reasons: They can effectively attach the cells to the filmcarriers and proliferate them; and they hardly adsorb the desired biologically active substances produced by the cells, which may be therefore recovered efficiently.

Examples of preferred positively chargeable chemical moieties according to the present invention include ammonia; alkylamine such as ethylamine and butylamine; alkylenediamine such as ethylenediamine; dialkylamine such as diethylamine; alkoxyalkylamine such methoxyethylamine and ethoxyethylamine; alkanolamine such as ethanolamine, 2-amino-1-butanolamine, N-methylethanolamine.

The above-mentioned chemical moieties may be introduced on the surface of the filmcarriers of the present invention by stirring the water-insoluble polymer film obtained in the above manner and, for example, the above amines in a suitable solvent such as water, ethanol, tetrahydrofuran, 1,4-dioxane for a few hours at a temperature ranging between a room temperature and a refluxing point of the solvent. The film gel may be chemically modified with proteins such as gelatin and collagen by means of cyanogen bromide, carbonyl imidazole or glutaraldehyde.

In view of the aspects of the present invention, it is at least required that the positively chargeable chemical moieties or proteins should be present in a layer ranging from the film gel surface to about 10 nm deep in the film gel, which layer may be in contact with the cells. The positively chargeable chemical moieties or proteins may, however, be present more deep inside the film gel without causing any deleterious effect.

The content of the positively chargeable chemical moieites or proteins may vary with such factors as the cells, the chemical moieties or proteins themselves, the concentration of filmcarriers in culture medium and the culture conditions. The charge capacity found suitable ranges between 0.1 and 4.0 meq per one gram of dry filmcarriers.

The present invention is further illustrated by the following examples, which may not be construed as limiting the scope of this invention.


EXAMPLE 1


The filmcarriers according to the present invention were prepared as follows:

There was prepared a monomer solution containing 4.00 g of polyethyleneglycol (meth)acrylate (PE-350: trade name, commercialy available from Nippon Oil and Fats Co., Ltd.), 3.00 g of glycidyl (meth)acrylate, 3.00 g of nonaethyleneglycol di(meth)acrylate, 6.0 g of 1-hexanol, 4.0 g of cyclohexanol, 50 mg of 2,2-azobis-(2,4-dimethyl-4-methoxyvaleronitrile (V-70, trade name) as a polymerization initiator and 0.2 ml of methylene chloride. Into this solution was introduced $N_2$ gas to remove out a dissolved oxygen.

The resulting solution was poured into a cell consisting of a pair of glass plates spaced from each other with a 25 $\mu$m spacer. The cell was then heated for 10 min. at 70°C to give film gel. After the completion of polymerization, the film gel was removed from the cell and washed with 1,4-dioxane to yield a colorless and transparent film gel, which is referred to hereinafter as "Polymer 1". Polymer 1 having a total surface area of 5,000 cm² was taken into 200 ml of 1,4-dioxane. To this was added dropwise 20 ml of 1,4-dioxane solution containing 15.0 g of ethanolamine. After the reaction with stirring for 4 hours at 75°C, the resulting film gel was washed thoroughly with water, which is referred to hereinafter as "Filmcarrier-1 (FC-1)". The elemental analysis of FC-1 is as follows:

| C | H | N |
|---|---|---|
| 52.35% | 8.06% | 2.67% . |

Based on the above analysis the charge capacity of FC-1 was characterized to be 2.15 meq per gram of dry gel.


EXAMPLE 2


Polymer 1 having a total surface area of 5,000 cm² was taken into 200 ml of 1,4-dioxane. To this was added dropwise 20 ml of 1,4-dioxane solution containing 15.0 g of methoxyethylamine. After the reaction with stirring for 4 hours at 75°C, the resulting film gel was washed thoroughly with water, which is referred to hereinafter as "Filmcarrier-2 (FC-2)". The elemental analysis of FC-2 is as follows:

| C | H | N |
|---|---|---|
| 51.68% | 8.21% | 2.54% . |

Based on the above analysis the charge capacity of FC-2 was characterized to be 2.05 meq per gram of dry gel.

## EXAMPLE 3

There was prepared at a low temperature a monomer solution containing 4.30 g of polyethyleneglycol (meth)acrylate (PE-90): trade name, commecialy available from Nippon Oil and Fats Co., Ltd.), 2.70 g of glycidyl (meth)acrylate, 3.00 g of tetraethyleneglycol di(meth)acrylate, 10.0 g of cyclohexanol, 50 mg of 2,2-azobis-(2,4-dimethyl-4-methoxyvaleronitrile (V-70, trade name) as a polymerization initiator and 0.30 ml of methylene chloride. Into this solution was introduced $N_2$ gas to remove out a dissolved oxygen.

The resulting solution was poured into a cell consisting of a pair of glass plates spaced from each other with a 120 μm spacer. The cell was then heated for 10 min. at 70°C to give film gel. After the completion of polymerization, the film gel was removed from the cell and washed with 1,4-dioxane to yield a colorless and transparent film gel, which is referred to hereinafter as "Polymer 2". Polymer 2 having a total surface area of 5,000 cm² was taken into 200 ml of 1,4-dioxane. To this was added dropwise 20 ml of 1,4-dioxane solution containing 15.0 g of diethylamine. After the reaction with stirring for 4 hours at 75°C, the resulting film gel was washed thoroughly with water, which is referred to hereinafter as "Filmcarrier-3 (FC-3)". The elemental analysis of FC-3 is as follows:

| C | H | N |
|---|---|---|
| 51.36% | 8.07% | 2.92% . |

Based on the above analysis the charge capacity of FC-3 was characterized to be 2.42 meq per gram of dry gel.

## EXAMPLE 4

There was prepared a monomer colution containing 3.70 g of polyethyleneglycol (meth)acrylate (PE-200: trade name, commecialy available from Nippon Oil and Fats Co., Ltd.), 3.30 g of glycidyl (meth)acrylate, 3.00 g of glycerol di(meth)acrylate, 4.0 g of cyclohexanol, 6.0 g of 1-hexanol, 50 mg of 2,2-azobis-(2,4-dimethyl-4-methoxyvaleronitrile (V-70, trade name) as a polymerization initiator and 0.30 ml of methylene chloride. Into this solution was introduced $N_2$ gas to remove out a dissolved oxygen.

The resulting solution was poured into a cell consisting of a pair of glass plates spaced form each other with a 120 μm spacer. The cell was then heated for 10 min. at 70°C to give film gel. After the completion of polymerization, the film gel was removed from the cell and washed with 1,4-dioxane to yield a colorless and transparent film gel, which is referred to hereinafter as "Polymer 3". Polymer 3 having a total surface area of 5,000 cm² was taken into 500 ml of 1,4-dioxane. To this was added dropwise 20 ml of 1,4-dioxane solution containing 18.0 g of N-methylethanolamine. After the reaction for 4 hours at 75°C with stirring, the resulting film gel was washed thoroughly with water, which is referred to hereinafter as "Filmcarrier-4 FC-4)". The elemental analysis of FC-4 is as follows:

| C | H | N |
|---|---|---|
| 51.88% | 7.95% | 3.06% . |

Based on the above analysis the charge capacity of FC-4 was characterized to be 2.60 meq per gram of dry gel.

EXAMPLE 5

FC-1 having a total surface area of 1,000 cm$^2$ was taken into 100 ml of 0.5 % gelatin aqueous solution and then stirred. To this was slowly added dropwise 1.0 ml of 30 % glutaraldehyde aqueous solution and the resulting mixture was stirred for about 30 min. After the reaction was completed, the resulting filmcarriers were washed thoroughly with water, which is referred to hereinafter as "Filmcarrier-5 (FC-5)".

EXAMPLE 6

Vero cells (African green monkey's renal cells) and CHO-K$_1$ cells (Chinese Hamster Ovary) cells, both of which are anchorage-dependent cells, were subjected to cultivation using the cell culture filmcarriers according to the present invention.

FC-1, FC-2, FC-3, FC-4 and FC-5 were thoroughly washed with phosphate buffer (pH 7.40) and Eagle's MEM successively. The filmcarriers thus washed were autoclaved at 121°C for 30 min. prior to the use in cultivation. The amount of the filmcarriers in a culture medium may depend on the thickness of the filmcarriers, that of from 5 to 50 % of the medium being actually used.

The cell culture was performed in the following two procedures:

1. The filmcarriers (25 μm in thickness and 1 m$^2$ of total surface area) sterilized as the above (20 ml), 250 ml of TS-2 incubated at 37°C and 15.0 ml of fetal calf serum were added to a sterilized spinner flask. To this was supplemented 20 ml of TS-2 suspension containing 1 - 3 x 10$^7$ Vero or CHO cells. The culture was carried out at 37°C under 5 % CO$_2$ with a continuous agitation. At day 6 or 7, a part of the medium (250 ml) was replaced with a fresh medium (250 ml) and serum (15.0 ml). The cells had reached an almost confluent state in about 6 - 10 days. After the culture medium was exchanged with a serum-free one, the culture was then continued with a daily exchange of the culture medium.

2. Into a sterilized column (500 ml) 40 ml of the filmcarriers, 350 ml of a medium, 25 ml of serum and 50 ml of a TS-2 suspension containing 2 - 5 x 10$^7$ Vero or CHO cells. The culture was carried out in a flowing state while circulating the medium in an up-flow fashion. At days 5 - 7, whole medium was exchanged with 350 ml of TS-2 and 40 ml of serum. After the medium was completely exchanged with a serum-free one at days 6 -10, the culture was then continued with a daily exchange of the culture medium.

The results obtained in the above cultures are shown in TABLE 1 below.

TABLE 1

| Filmcarrier | degree of cell attachment | degree of cell growth |
|---|---|---|
| FC-1 | very high | high |
| FC-2 | very high | very high |
| FC-3 | very high | very high |
| FC-4 | very high | very high |
| FC-5 | very high | not so high |

In the cases of FC-1 and FC-2, FC-3, and FC-4, the results were obtained using CHO cells at day 12, day 4, and day 3, respectively. On the other hand, the result concerning FC-5 was obtained at day 3 using Vero cells.

EXAMPLE 7

FC-3 was washed with phospate buffer (pH 7.40) and Eagle's MEM successively followed by sterilization in an autoclave at 121°C for 20 min.

The resulting FC-3 (100 cm$^2$ of total surface area and 1 μm in thickness), 20 ml of TS-2 medium and 2.0 ml of fetal calf serum were taken into a sterilized T-flask. Incubation was started by adding $5 \times 10^6$ cells of hybridomas to the T-flask. From day 3 of culture, the medium was exchanged on every day, during which medium exchanges no detachment of the hybridomas was observed. The degree of cell attachment and cell growth at day 5 were high and very high, respectively.

**Claims**

1. Cell culture filmcarriers consisting of water-insoluble polymer film, the film being 1.0 - 2,000 $\mu$m in thickness and 1.00 - 1.30 g/ml in density in a culture medium and having positively chargeable chemical moieties or proteins, which are linked on the film surface through at least chemical bonds.

2. Cell culture filmcarriers according to claim 1, wherein the water-insoluble polymer is constituted by (meth)acrylate ester.

3. Cell culture filmcarriers according to claim 1, wherein the charge capacity is adjusted to the range of between 0.1 and 4.0 meq per gram of dry gel.

4. Cell culture filmcarriers according to claim 1, wherein the positively chargeable chemical moieties are ammonia or amine having 5 or less carbon atoms.

5. Cell culture filmcarriers according to claim 1, wherein the proteins are collagen or gelatin.